# EUROPEAN PATENT SPECIFICATION

(11) **EP 1 629 772 B1**
(45) Date of publication and mention of the grant of the patent: **22.10.2008**
(21) Application number: 05018843.2
(22) Date of filing: 30.08.2005
(51) Int. Cl.: A61B 5/053

(54) **Body composition meter adapted for children**
Kindergeeignetes Körperzusammensetzungsmessgerät
Appareil de mesure de la composition corporelle adapté aux enfants

(30) Priority: 31.08.2004 JP 2004252989; 10.09.2004 JP 2004263173
(43) Date of publication of application: 01.03.2006
(73) Proprietor: TANITA CORPORATION, Tokyo (JP)
(72) Inventor: Takehara, Tomoko c/o TANITA CORPORATION, Tokyo (JP)
(74) Representative: Grünecker, Kinkeldey, Stockmair & Schwanhäusser Anwaltssozietät

(56) References cited:
- WO-A-03/046493
- US-A1- 2003 216 665
- US-A1- 2004 035 611
- US-A1- 2004 148 127
- US-B1- 6 354 996

## Description

### BACKGROUND OF THE INVENTION

### (i) Field of the Invention

The present invention relates to a body composition meter having a function of determining the body compositions of children which measures a value associated with the body composition (which is generally used as a generic term for body fat, visceral fat, subcutaneous fat, muscles, bone, body water and other components in the human body) of children who are in a significant growth period in the growing process of human beings and determines a proper grade for the measured value associated with the body composition.

### (ii) Description of the Related Art

Heretofore, a number of apparatuses which measure a living body have been introduced to the market from the viewpoint of health consciousness. Further, in recent years in particular, apparatuses capable of measuring a value associated with body fat, visceral fat or the like and determining a proper grade for the measured value associated with body fat, visceral fat or the like have been increasingly introduced to the market, since obesity may cause lifestyle-related diseases.

Examples of such apparatuses are disclosed in the following Patent Publications.

An apparatus disclosed in Patent Publication 1 measures a body fat area and determines which determination area the measured body fat area belongs to.

An apparatus disclosed in Patent Publication 2 measures a body fat percentage and determines which determination area the measured body fat percentage belongs to, calculates a BMI and determines which determination area the calculated BMI belongs to, and makes an overall determination on which determination area both of these determination results belong to.

An apparatus disclosed in Patent Publication 3 calculates a BMI, a body fat percentage and an abdominal visceral fat cross-sectional area from measured and entered data and determines which determination area each of the calculated BMI, body fat percentage and abdominal visceral fat cross-sectional area belongs to.

These apparatuses are very convenient and useful for health management because users can not only acquire a result such as a body fat area but also know a proper grade for the result by use of these apparatuses.
Patent Publication 1
Japanese Patent Laid-Open Publication No. 2002-191563
Patent Publication 2
Japanese Patent Laid-Open Publication No. 2002-191573
Patent Publication 3
Japanese Patent Laid-Open Publication No. 2003-52659

However, determination standards in the above apparatuses are differentiated merely by sex, i.e. males and females, and are intended for both children and adults or for adults only. The determination standards intended for both children and adults lack reliability because children and adults are different in the level of growth and borderlines for proper grades should be different accordingly. Meanwhile, the determination standards intended for adults only are inconvenient because determinations cannot be made on infants and children with the determination standards.

Under the above circumstances, an object of the present invention is to provide a body composition meter having a function of determining the body compositions of children by which a subject can conveniently acquire highly reliable data about determination of a proper grade for the body composition of the child.

US 6,534,996 B discloses a body composition meter comprising a platform with foot electrodes, means for determining the impedance of the body, a body weight measuring unit, a microcomputer for determining the body composition on the basis of the bioelectrical impedance, of the weight and of a growth indicator such as height or age.

### SUMMARY OF THE INVENTION

To achieve the above object, according to one aspect, a body composition meter having a function of determining the body compositions of children according to the present invention comprises the features of claim 1.

### BRIEF DESCRIPTION OF THE DRAWINGS

Fig. 1 is external views of a body composition meter with a function of determining the body compositions of children according to the present invention, wherein A is a plan view thereof, B is a side view thereof, and C is a front view thereof.
Fig. 2 is a block diagram illustrating the overall constitution of the body composition meter with a function of determining the body compositions of children according to the present invention.
Fig. 3 is a flowchart showing a flow of the operations of the body composition meter with a function of determining the body compositions of children according to the present invention.
Fig. 4 is a diagram showing age-based body fat percentage determination reference tables, wherein A is a table for boys and B is a table for girls.
Fig. 5 is a diagram showing age-based body fat percentage determination graph data, wherein A is data for boys and B is data for girls.
Fig. 6 is a diagram showing age-based fat free percentage determination reference tables, wherein A is a table for boys and B is a table for girls.
Fig. 7 is a diagram showing age-based fat free percentage determination graph data, wherein A is data for boys and B is data for girls.
Fig. 8 is a diagram showing body-height-based body fat percentage determination graph data, wherein A is data for boys and B is data for girls.
Fig. 9 is a diagram showing a display section displaying a screen for setting personal data.
Fig. 10 is a diagram showing the display section displaying a screen for displaying results such as measurement data.
Fig. 11 is a weekly basis history graph.
Fig. 12 is a monthly basis history graph.
Fig: 13 is a yearly basis history graph.
Fig. 14 is a diagram showing the display section displaying the screen for displaying results such as measurement data in another mode.
Fig. 15 is a diagram showing the display section displaying the screen for displaying results such as measurement data in still another embodiment.
Fig. 16 is a diagram showing other age-based body fat percentage determination reference tables, wherein A is a table for boys and B is a table for girls.
Fig. 17 is a diagram showing other age-based body fat percentage determination graph data, wherein A is data for boys and B is data for girls.
Fig. 18 is a diagram showing other age-based fat free percentage determination reference tables, wherein A is a table for boys and B is a table for girls.
Fig. 19 is a diagram showing other age-based fat free percentage determination graph data, wherein A is data for boys and B is data for girls.
Fig. 20 is a diagram showing other body-height-based body fat percentage determination graph data, wherein A is data for boys and B is data for girls.

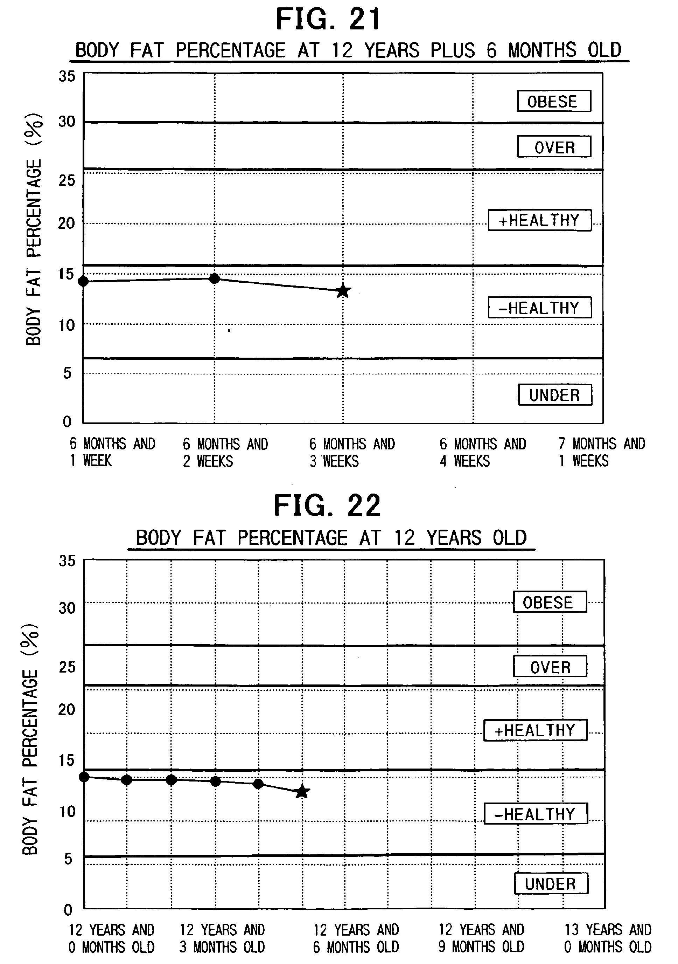

Fig. 21 is another weekly basis history graph.
Fig. 22 is another monthly basis history graph.
Fig. 23 is another yearly basis history graph.
Fig. 24 is a diagram showing a part of the display section of another embodiment, displaying a screen for displaying results such as measurement data.
Fig. 25 is a diagram showing other age-based body fat percentage determination reference tables, wherein A is a table for boys and B is a table for girls.

### DETAILED DESCRIPTION OF THE PREFERRED EMBODIMENTS

As a first embodiment, a body composition meter having a function of determining the body compositions of children according to the present invention comprises determination object selecting means, body composition acquiring means, growth indicator acquiring means, child body composition determination standard storing means, child body composition determining means, determination result displaying means, and abrupt change warning means. The terms "child" and "children" used in the description of the present specification refer to those of age 17 or younger. Further, the term "acquiring" used herein refers to acquiring by input and measurement, acquiring solely by input or acquiring solely by measurement.

The determination object selecting means selects a value (e.g., a body fat percentage, a fat free percentage or a muscle percentage) associated with the body composition of a child which is to be acquired by the body composition acquiring means.

The body composition acquiring means acquires the value (e.g., a body fat percentage, a fat free percentage or a muscle percentage) associated with the body composition of the child selected by the determination object selecting means.

The growth indicator acquiring means acquires an indicator (e.g., age or a body height) indicating the level of the growth of the child.

The child body composition determination standard storing means stores a body composition determination standard showing proper grades for values (e.g., a body fat percentage, a fat free percentage or a muscle percentage) associated with the body composition of children as a plurality of ranges according to an indicator (e.g., age or a body height) indicating the level of the growth of children based on an obesity determination standard showing proper grades for values (e.g., the degree of obesity, a BMI (Body Mass Index) or a Rohrer index) associated with the obesity of children as a plurality of ranges.

The child body composition determining means determines a proper grade for the value (e.g., a body fat percentage, a fat free percentage or a muscle percentage) associated with the body composition of the child which corresponds to the value acquired by the body composition acquiring means and the indicator acquired by the growth indicator acquiring means by referring to the body composition determination standard stored in the child body composition determination standard storing means.

The determination result displaying means displays the result of the proper grade for the value associated with the body composition of the child determined by the child body composition determining means. Further, the determination result displaying means also displays history graphs in turn, the history graphs each comprising a time axis and a body composition value axis, the history graphs showing the current value associated with the body composition of the child together with past values associated with the body composition of the child such that the current value is positioned in the center of the time axis, one history graph showing a transition of the values by weeks, another history graph showing the transition of the values by months, the other history graph showing the transition of the values by years.

The abrupt change warning means gives a warning when the amount of change between the current value associated with the body composition of the child acquired by the body composition acquiring means and the past values associated with the body composition of the child is larger than a predetermined range value. The term "predetermined range value" refers to a range value at which a change in value associated with body composition may occur in a normal growth.

According to the thus constituted body composition meter having a function of determining the body compositions of children, the child body composition determining means determines a proper grade for a value associated with the body composition of a child which corresponds to the value associated with the body composition of the child acquired by the body composition acquiring means and an indicator indicating the level of the growth of the child acquired by the growth indicator acquiring means by referring to a body composition determination standard (which shows proper grades for values associated with the body composition of children as a plurality of ranges according to an indicator indicating the level of the growth of children based on an obesity determination standard showing proper grades for values associated with the obesity of children as a plurality of ranges) stored in the child body composition determination standard storing means. Thus, a subject can conveniently acquire highly reliable data about determination of a proper grade for the body composition of the child.

In the above description, when the value associated with obesity is a BMI (Body Mass Index) which is determined by an equation that takes only the body weight and body height of the child as parameters as shown by body weight (kg)/body height (m)², determination of the proper grade for the body composition of the child becomes highly reliable. When it is the degree of obesity which is determined by an equation that does not take parameters such as a body height which show a large individual difference in physical change in a growth period but takes only a body weight which reflects obesity as a parameter as shown by (body weight (kg) - standard body weight (kg))/standard body weight (kg) x 100, determination of the proper grade for the body composition of the child becomes particularly highly reliable.

Further, the determination object selecting means selects a value associated with the body composition of the child which is to be acquired by the body composition acquiring means, and the body composition acquiring means acquires the selected value associated with the body composition of the child. Thus, the degree of freedom in desired determination is increased, and data about the determination can be acquired more conveniently.

Further, the determination result displaying means displays history graphs in turn,
the history graphs each comprising a time axis and a body composition value axis,
the history graphs showing the current value associated with the body composition of the child together with past values associated with the body composition of the child such that the current value is positioned in the center of the time axis,
one history graph showing a transition of the values by weeks,
another history graph showing the transition of the values by months,
the other history graph showing the transition of the values by years. Thus, a subject can know the transition of the data more conveniently.

Further, the abrupt change warning means gives a warning when the amount of change between the current value associated with the body composition of a child and past values associated with the body composition of the child is larger than a predetermined range value. Thus, a subject can know an abrupt change in a body more conveniently.

As a second embodiment, a body composition meter having a function of determining the body compositions of children according to the present invention comprises determination object selecting means, body composition acquiring means, growth indicator acquiring means, child body composition determination standard storing means, body composition determination standard selecting means, child body composition determining means, determination result displaying means, and abrupt change warning means.

The growth indicator acquiring means acquires different indicators (e.g., age and a body height) indicating the level of the growth of the child.

The child body composition determination standard storing means stores a plurality of body composition determination standards showing proper grades for values associated with the body composition of children as a plurality of ranges according to an indicator (e.g., age or a body height) indicating the level of the growth of children based on an obesity determination standard showing proper grades for values (e.g., the degree of obesity, a BMI (Body Mass Index) or a Rohrer index) associated with the obesity of children as a plurality of ranges, for each of the different indicators (e.g., age and a body height) indicating the level of the growth of the child.

The body composition determination standard selecting means selects a body composition determination standard from the body composition determination standards stored in the child body composition determination standard storing means.

The child body composition determining means determines a proper grade for the value (e.g., a body fat percentage, a fat free percentage or a muscle percentage) associated with the body composition of the child which corresponds to the value (e.g., a body fat percentage, a fat free percentage or a muscle percentage) acquired by the body composition acquiring means and the indicator (e.g., age or a body height) corresponding to the body composition determination standard selected by the body composition determination standard selecting means out of the different indicators acquired by the growth indicator acquiring means, by referring to the body composition determination standard selected by the body composition determination standard selecting means.

Descriptions of the determination object selecting means, the body composition acquiring means, the determination result displaying means and the abrupt change warning means will be omitted since these are the same as those in the first embodiment.

According to the thus constituted body composition meter having a function of determining the body compositions of children, the child body composition determining means determines a proper grade for the value associated with the body composition of the child which corresponds to the value acquired by the body composition acquiring means and the indicator corresponding to the body composition determination standard selected by the body composition determination standard selecting means out of the different indicators acquired by the growth indicator acquiring means, by referring to the body composition determination standard (which shows proper grades for values associated with the body composition of children as a plurality of ranges according to an indicator indicating the level of the growth of children based on an obesity determination standard showing proper grades for values associated with the obesity of children as a plurality of ranges) selected by the body composition determination standard selecting means.

Hereinafter, an example (body composition meter with a function of determining the body compositions of children of ages 6 to 17) in the above embodiment will be described specifically.

### Examples

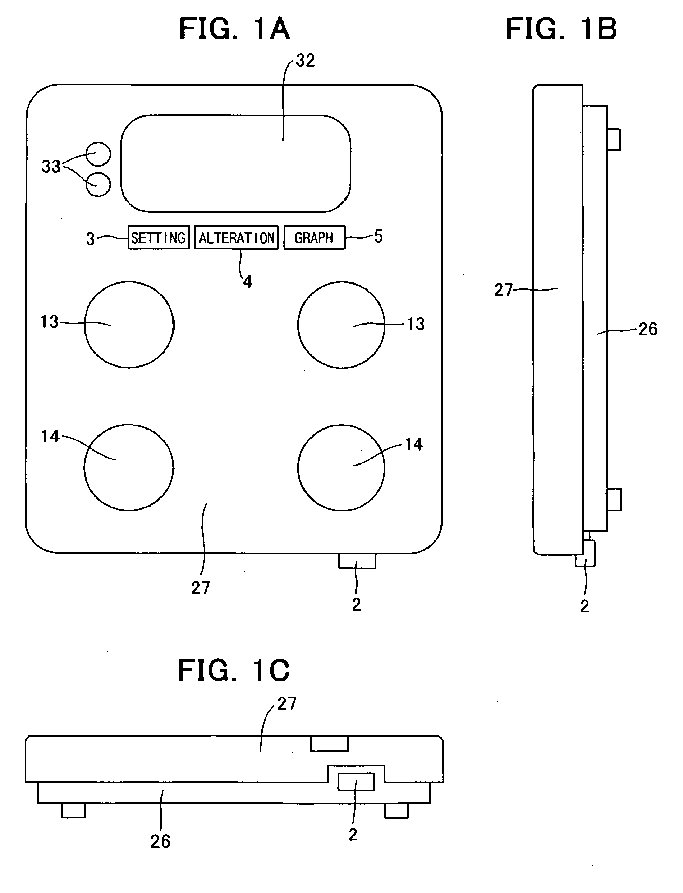

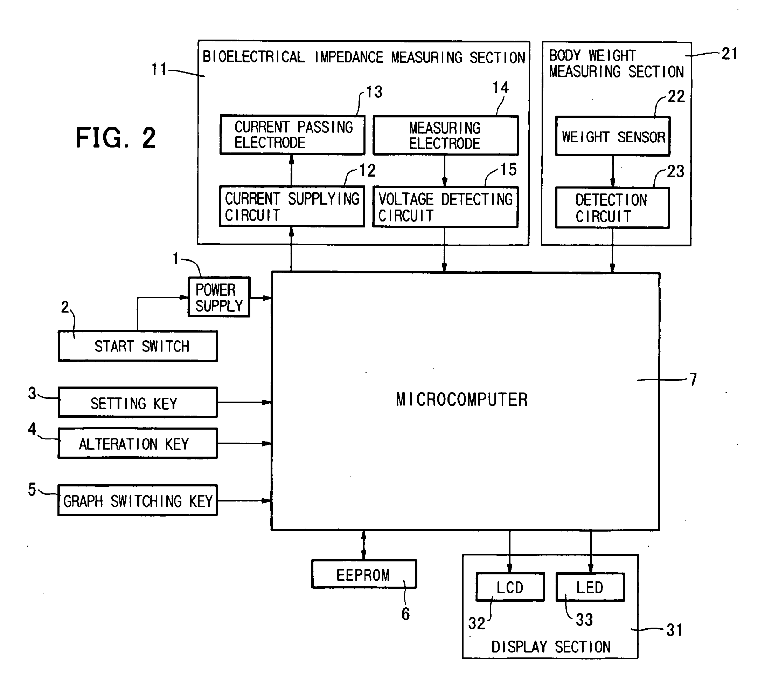

First, the specific constitution of a body composition meter with a function of determining the body compositions of children according to the present invention will be described by use of an external view shown in Fig. 1 and a block diagram shown in Fig. 2.

The body composition meter according to the present invention comprises a power supply 1, a start switch 2, a setting key 3, an alteration key 4, a graph switching key 5, a bioelectrical impedance measuring section 11, a body weight measuring section 21, an EEPROM 6, a display section 31, and a microcomputer 7.

The power supply 1 is provided in a base 26 and supplies electric power to the sections constituting the electrical system of the present device.

The start switch 2 is provided on a side surface of the base 26 and supplies electric power from the power supply 1 to activate the present device.

The setting key 3 is provided on the top surface of a platform 27 and sets display of selected numerical values, characters or the like, storage of measurement data, and the like.

The alteration key 4 is provided on the top surface of the platform 27 and switches display of selected numerical values, characters or the like.

The graph switching key 5 is provided on the top surface of the platform 27 and switches among weekly basis, monthly basis and yearly basis history graphs to be displayed in the display section 31 (LCD 32) in turn.

The bioelectrical impedance measuring section 11 comprises a current supplying circuit 12, current passing electrodes 13, measuring electrodes 14 and a voltage detecting circuit 15 and measures a voltage generated based on a bioelectrical impedance. The current supplying circuit 12 is provided in the base 26 and generates a current to be passed through a body under the control of the microcomputer 7. The current passing electrodes 13 are provided on the top surface of the platform 27 so as to make contact with the bottoms of the feet, and a current is passed through the body from one current passing electrode 13 to the other current passing electrode 13. The measuring electrodes 14 are provided on the top surface of the platform 27 so as to make contact with the bottoms of the feet and detects a voltage generated based on a bioelectrical impedance in the body by the current passed from one current passing electrode 13 to the other current passing electrode 13. The voltage detecting circuit 15 is provided in the base 26 and amplifies and digitizes the detected voltage generated based on the bioelectrical impedance in the body.

The body weight measuring section 21 comprises a weight sensor 22 and a detection circuit 23 and measures a body weight when a subject stands on the platform 27. The weight sensor 22 is provided in the base 26 and detects a voltage generated based on the body weight when the subject stands on the platform 27. The detection circuit 23 is provided in the base 26 and amplifies and digitizes the detected voltage generated based on the body weight.

The EEPROM 6 is provided in the base 26 and stores measurement data set by means of the setting key 3 and various other data.

The display section 31 comprises an LCD 32 and LEDs 33. The display section 31 is provided on the top surface of the platform 27 and displays an input status, measurement results, warning messages and other data.

The microcomputer 7 comprises a CPU, a ROM which stores programs for control and computation, computing equations for computing a body fat percentage and a fat free mass, computing equations for computing the amount of change in the body fat percentage and the fat free mass, body composition determination reference tables and determination graph data, a RAM which stores computation results and personal data temporarily, a timer, and an I/O port. The microcomputer 7 is provided in the base 26 and executes processes such as control associated with determination of a proper grade for a value associated with the body composition of a child, determination of an abrupt change in body fat percentage or fat free mass and release of a warning about the abrupt change, and the like.

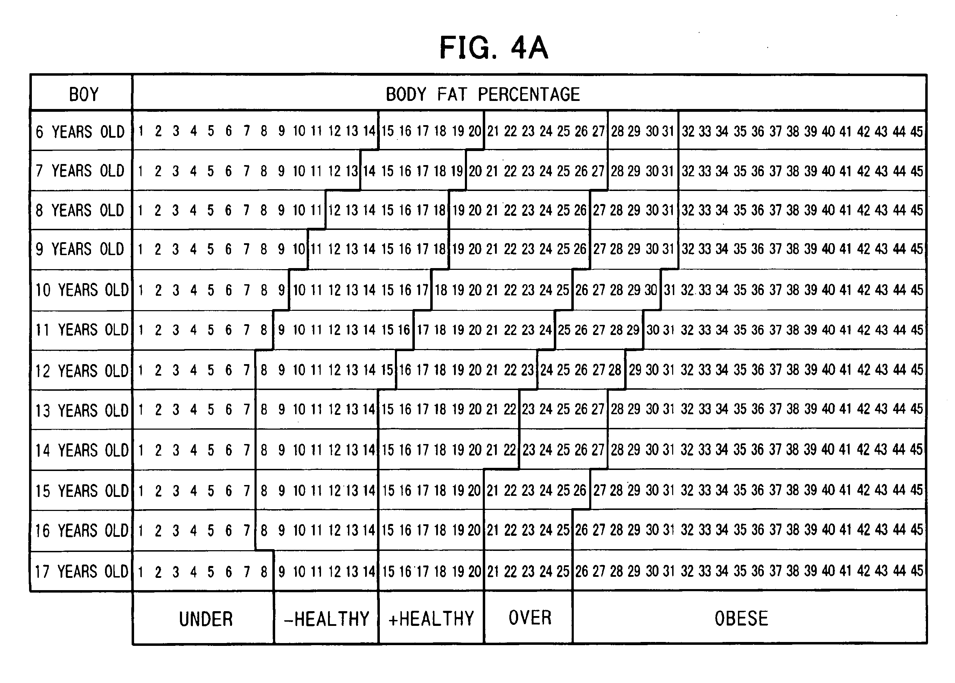

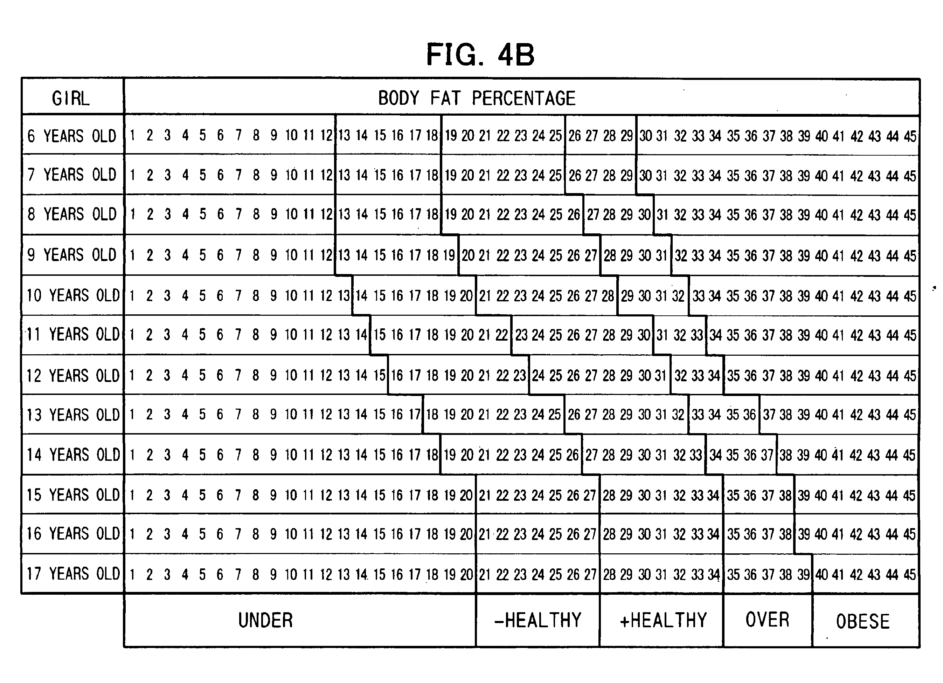

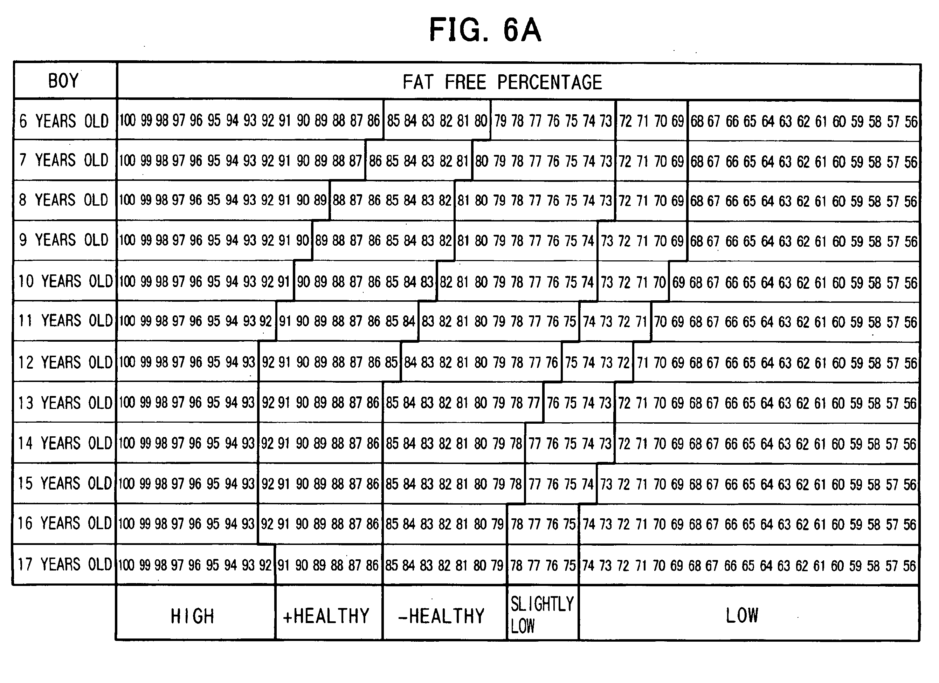

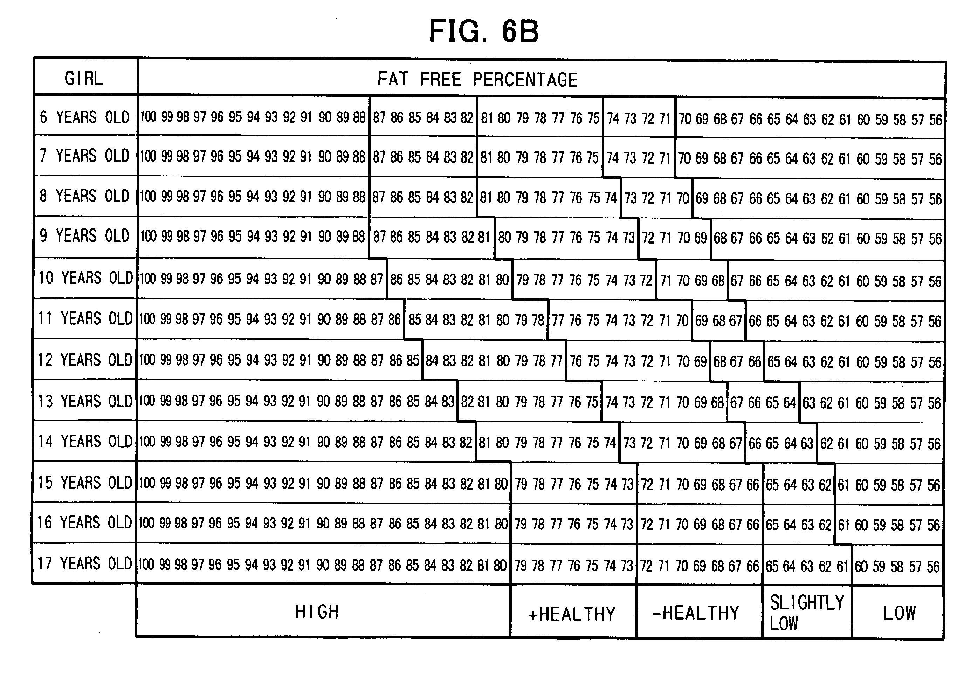

The body composition determination reference tables are a body fat percentage determination reference table showing proper grades (under, -healthy, +healthy, over, obese) for the body fat percentage of children as a plurality of ranges according to the ages of children based on an obesity determination standard showing proper grades for the degree of obesity (= (body weight (kg) - standard body weight (kg))/standard body weight (kg) x 100) of child as a plurality of ranges (refer to Fig. 4), a fat free percentage determination reference table showing proper grades (high, +healthy, -healthy, slightly low, low) for the fat free percentage of children as a plurality of ranges according to the ages of children based on the obesity determination standard showing the proper grades for the degree of obesity of child as a plurality of ranges (refer to Fig. 6), a body fat percentage determination reference table showing proper grades (under, -healthy, +healthy, over, obese) for the body fat percentage of children as a plurality of ranges according to the body heights of children based on the obesity determination standard showing the proper grades for the degree of obesity of child as a plurality of ranges (not shown), and a fat free percentage determination reference table showing proper grades (high, +healthy, -healthy, slightly low, low) for the fat free percentage of children as a plurality of ranges according to the body heights of children based on the obesity determination standard showing the proper grades for the degree of obesity of child as a plurality of ranges (not shown). As the obesity determination standard showing the proper grades for the degree of obesity of child as a plurality of ranges, a body shape determination standard (JASSO) based on the degree of obesity of schoolchildren which determines a child with a degree of obesity of +50 or higher to be highly obese, a child with a degree of obesity of not lower than +30% and lower than +50% to be moderately obese, a child with a degree of obesity of not lower than +20% and lower than +30% to be over, a child with a degree of obesity of not lower than -20% and lower than +20% to be healthy, and a child with a degree of obesity of lower than -20% to be under is used.

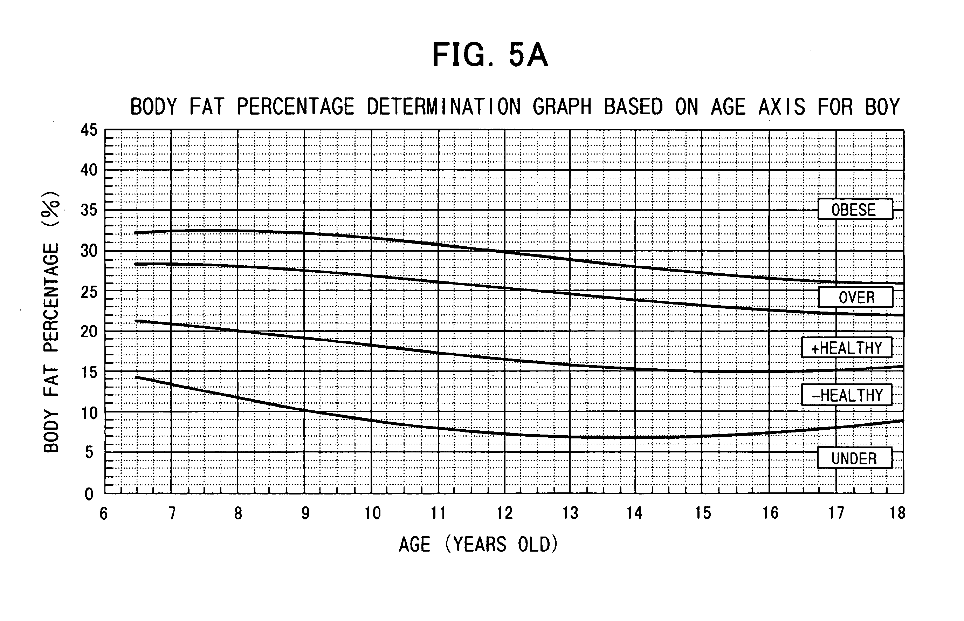

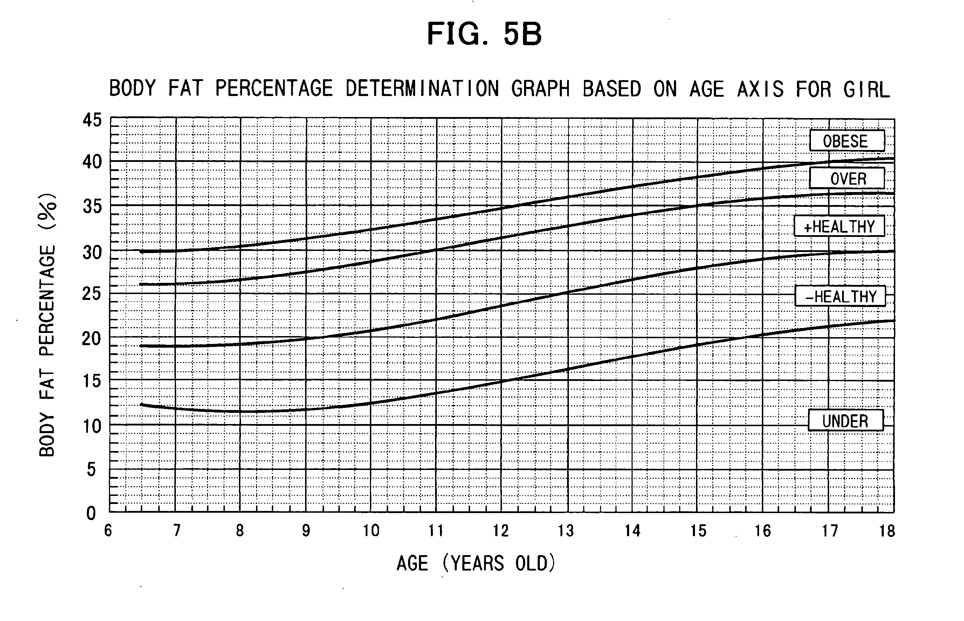

Further, the determination graph data are graphs of the above body composition determination reference tables (refer to Figs. 5, 7 and 8) .

The setting key 3, the alteration key 4 and the microcomputer 7 constitute determination object selectingmeans, body composition determination standard selecting means, and growth indicator acquiring means. The bioelectrical impedance measuring section 11 and the microcomputer 7 constitute body composition acquiring means. The microcomputer 7 constitutes child body composition determination standard storing means and child body composition determining means. The display section 31 and the microcomputer 7 constitute determination result displaying means and abrupt change warning means.

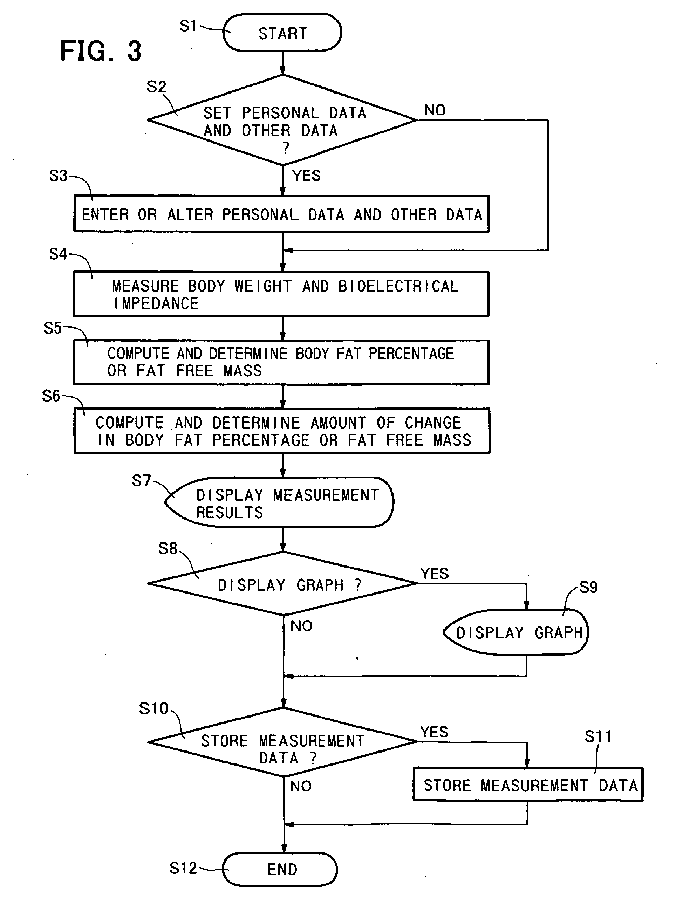

Next, the operation of the body composition meter having a function of determining the body compositions of children according to the present invention will be described by use of a flowchart shown in Fig. 3.

First, when a subject presses the start switch 2, the power supply 1 supplies electric power to the sections constituting the electrical system (STEP S1) so as to activate the device (STEP S2)

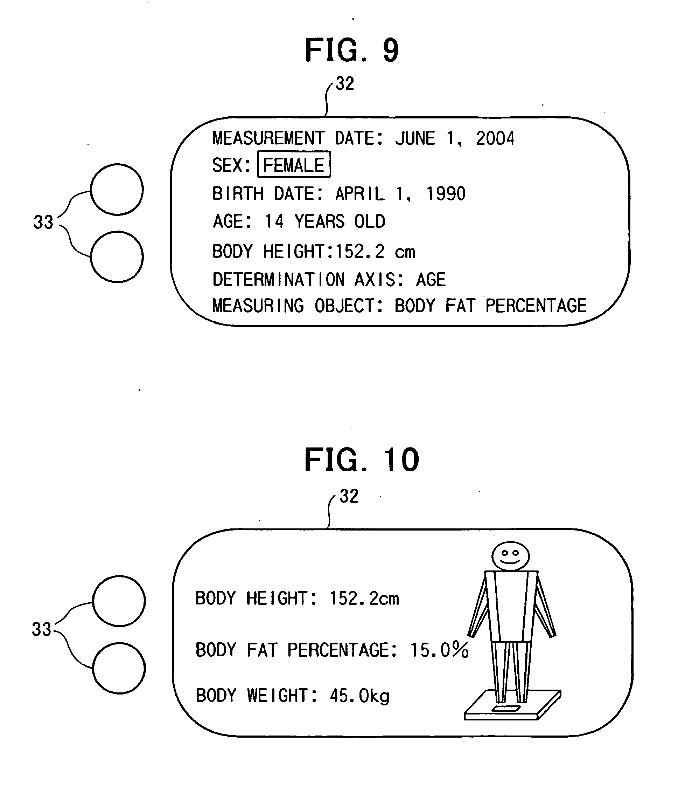

Then, when the subject presses the setting key 3 (YES in STEP S2), the LCD 32 displays a screen for setting personal data as shown in Fig. 9. Then, the subject alters and sets each of items (measurement date, sex, birth date, age, body height, determination axis, and measuring object) on the screen for setting personal data by use of the alteration key 4 and the setting key 3, and the EEPROM 6 stores each of the altered and set items (STEP S3). More specifically, when the screen for setting personal data is displayed, a cursor is positioned in the "measurement date" field first, and the date is altered by pressing the alteration key 4 and set by pressing the setting key 3. Then, the cursor moves to the "sex" field, and the sex is switched between a male and a female by pressing the alteration key 4 and set by pressing the setting key 3. Then, the cursor moves to the "birth date" field, and the birth date is altered by pressing the alteration key 4 and set by pressing the setting key 3. Then, the cursor moves to the "age" field, and the age is altered within a range of 6 to 17 years old by pressing the alteration key 4 and set by pressing the setting key 3. Then, the cursor moves to the "body height" field, and the body height is altered by pressing the alteration key 4 and set by pressing the setting key 3. Then, the cursor moves to the "determination axis" field, and the determination axis is switched between age and a body height by pressing the alteration key 4 and set by pressing the setting key 3. Then, the cursor moves to the "measuring object" field, and the measuring object is switched between a body fat percentage and a fat free percentage by pressing the alteration key 4 and set (i.e. stored in the EEPROM 6) by pressing the setting key 3. Thereby, the screen for setting personal data is switched to a measurement screen.

Then, when the subject does not press the setting key 3 in STEP S2 (NO in STEP S2) or stands on the platform 27 after STEP S3, the body weight and bioelectrical impedance of the subject are measured in the body weight measuring section 21 and the bioelectrical impedance measuring section 11, respectively (STEP S4), and in the microcomputer 7, current measurement data of the measuring object (body fat percentage or fat free percentage) set on the screen for setting personal data is calculated by use of the computing equation for computing a body fat percentage or a fat free percentage based on the above measured body weight and bioelectrical impedance. Then, in the microcomputer 7, a proper grade for the current measurement data (body fat percentage or fat free percentage) is determined by use of a body composition determination reference table corresponding to the sex, determination axis and measuring object set on the screen for setting personal data (i.e. one of an age-based body fat percentage determination reference table for males, an age-based body fat percentage determination reference table for females, an age-based fat free percentage determination reference table for males, an age-based fat free percentage determination reference table for females, a body-height-based body fat percentage determination reference table for males, a body-height-based body fat percentage determination reference table for females, a body-height-based fat free percentage determination reference table for males, and a body-height-based fat free percentage determination reference table for females) (STEP S5).

Then, in the microcomputer 7, the difference between the above-calculated current measurement data (body fat percentage or fat free percentage) and past measurement data (average of past data on a month ago) stored in the EEPROM 6 is calculated, and it is determined whether the difference is larger than a predetermined range value (standard deviation value (1SD) for the average of past data on a month ago) (STEP S6).

Then, as shown in Fig. 10, the LCD 32 displays some of the items set on the screen for setting personal data and the current measurement data. The upper LED 33 lights in color corresponding to the above determined proper grade for the current measurement data (body fat percentage or fat free percentage) (i.e. blue for under or high, yellowish green for -healthy, green for +healthy, orange for over or slightly low, and red for obese or low), and the lower LED 33 blinks in red only when the above determined difference is larger than the standard deviation value (1SD) (STEP S7).

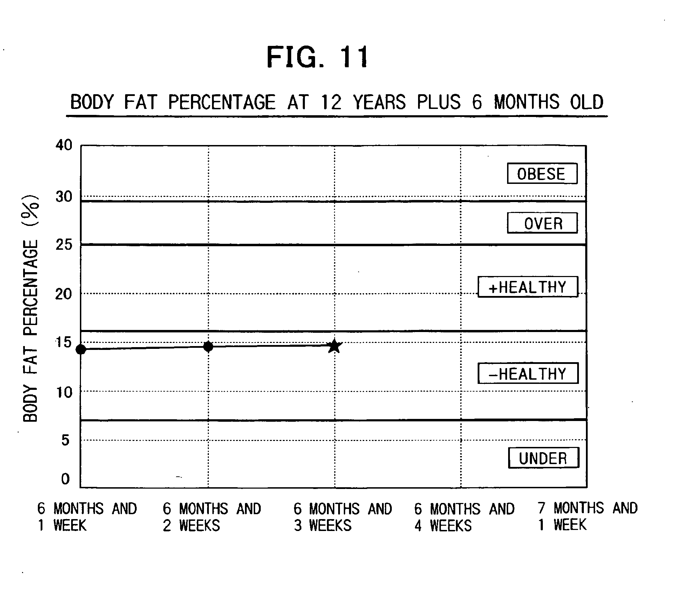

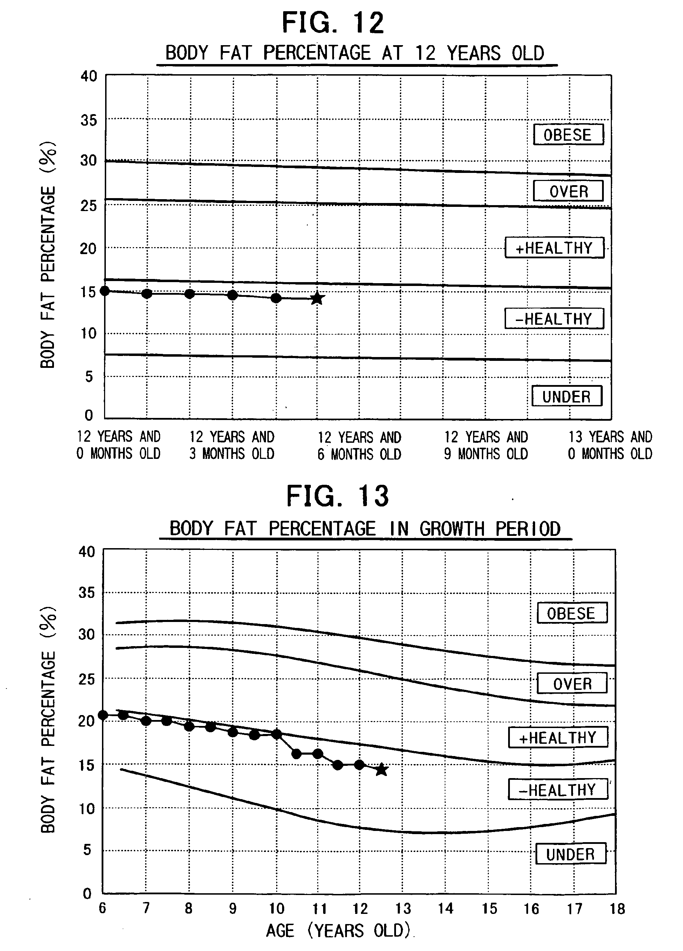

Then, with the LCD 32 displaying some of the items set on the screen for setting personal data and the current measurement data (STEP S8), when the graph switching key 5 is pressed (YES in STEP S8), the LCD 32 displays the current measurement data in the center of the time axis on the screen, in a history graph whose graduation lines represent weeks, the history graph showing measurement data averaged by weeks based on stored past measurement data and the current measurement data and showing proper grades for the measurement data as a plurality of ranges based on determination graph data, as shown in Fig. 11. Then, when the graph switching key 5 is pressed, the LCD 32 displays the current measurement data in the center of the time axis on the screen, in a history graph whose graduation lines represent months, the history graph showing measurement data averaged by months based on the stored past measurement data and the current measurement data and showing the proper grades for the measurement data as a plurality of ranges based on the determination graph data, as shown in Fig. 12. Then, when the graph switching key 5 is pressed, the LCD 32 displays the current measurement data in the center of the time axis on the screen, in a history graph whose graduation lines represent years, the history graph showing measurement data averaged by years based on the stored past measurement data and the current measurement data and showing the proper grades for the measurement data as a plurality of ranges based on the determination graph data, as shown in Fig. 13 (STEP S9).

Then, when the graph switching key 5 is not pressed in STEP S8 (NO in STEP S8) or when the setting key 3 is pressed (YES in STEP S10) after STEP S9 (STEP S10), the EEPROM 6 stores the current measurement data as past measurement data continuously (STEP S11).

Then, after a certain period of time has elapsed without pressing the graph switching key 5 (NO in STEP S10) or after STEP S11, the power supply 1 stops supplying electric power to the sections constituting the electrical system, thereby deactivating the device (STEP S12).

An example of the body composition meter with a function of determining the body compositions of children according to the present invention is constituted as described above.

In the above example, the various body composition determination reference tables are based on the obesity determination standard showing proper grades for the degree of obesity of child as a plurality of ranges. However, the body composition determination reference tables may also be based on an obesity determination standard showing proper grades for the BMI (= body weight (kg)/body height (m)²) of child as a plurality of ranges or an obesity determination standard showing proper grades for the Rohrer index (= body weight (kg) /(body height (cm)³) x 10⁷) of child as a plurality of ranges.

Further, in the above example, the measuring objects are a body fat percentage and a fat free percentage. However, the measuring object may also be a muscle percentage.

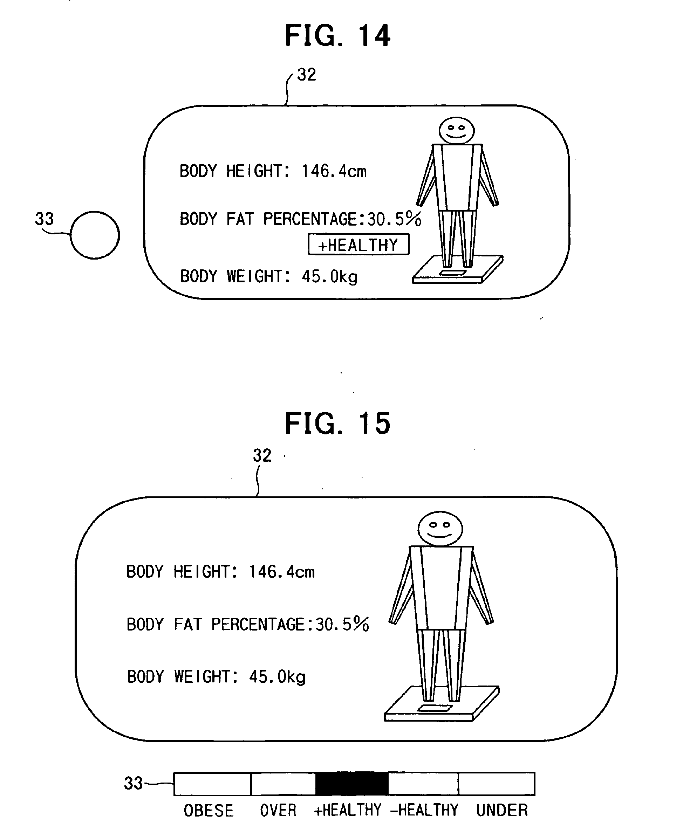

Further, the result of determination of the proper grade for the current measurement data is indicated by lighting the upper LED 33 in color corresponding to the proper grade as shown in Fig. 10. In addition, it is also possible to display the corresponding proper grade on the LCD 32 together with some of the items set on the screen for setting personal data and the current measurement data as shown in Fig. 14, to light the corresponding section in a bar graph sectioned according to the proper grades as shown in Fig. 15, or to sound a buzzer.

Further, in the above example, the body shape determination standard (JASSO) based on the degree of obesity of schoolchildren is used as the obesity determination standard showing proper grades for the degree of obesity of child as a plurality of ranges, because the present device is intended for children of ages 6 to 17. However, in the case of a body composition meter having a function of determining the body compositions of children of age 6 or younger, a body shape determination standard (JASSO) for small children (age 6 or younger) which determines the degree of obesity on the basis of +15% can be used, for example.

Further, in the above example, age is entered when the data are entered or altered on the screen for setting personal data (STEP S3). However, it is also possible that age is not entered but calculated by the microcomputer from the entered measurement date and the entered birth date.

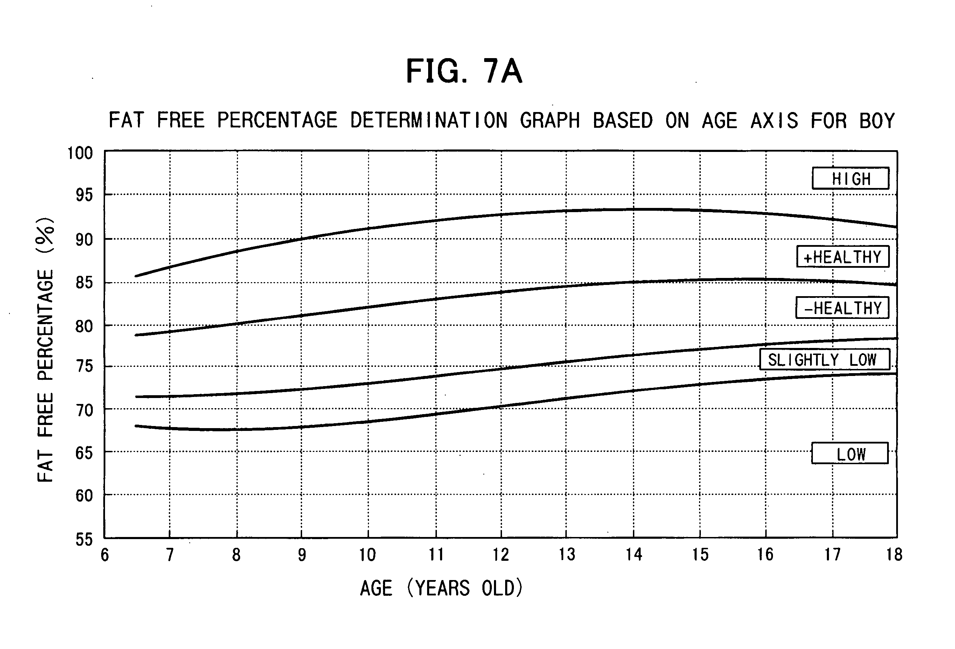

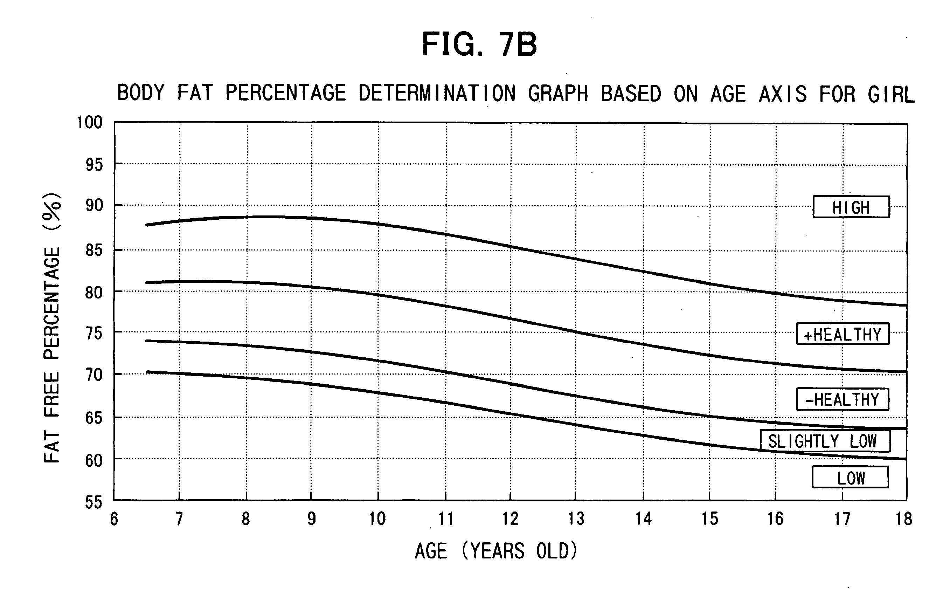

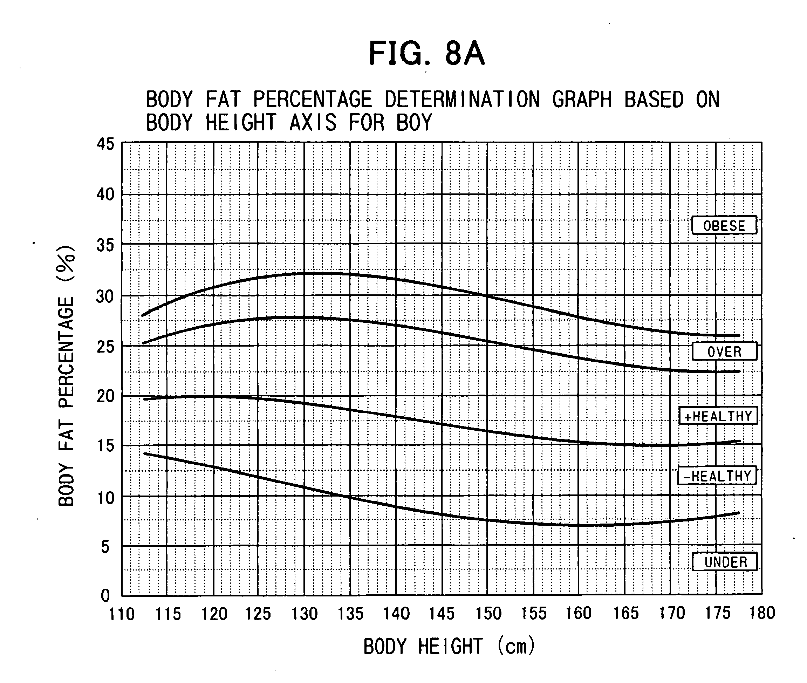

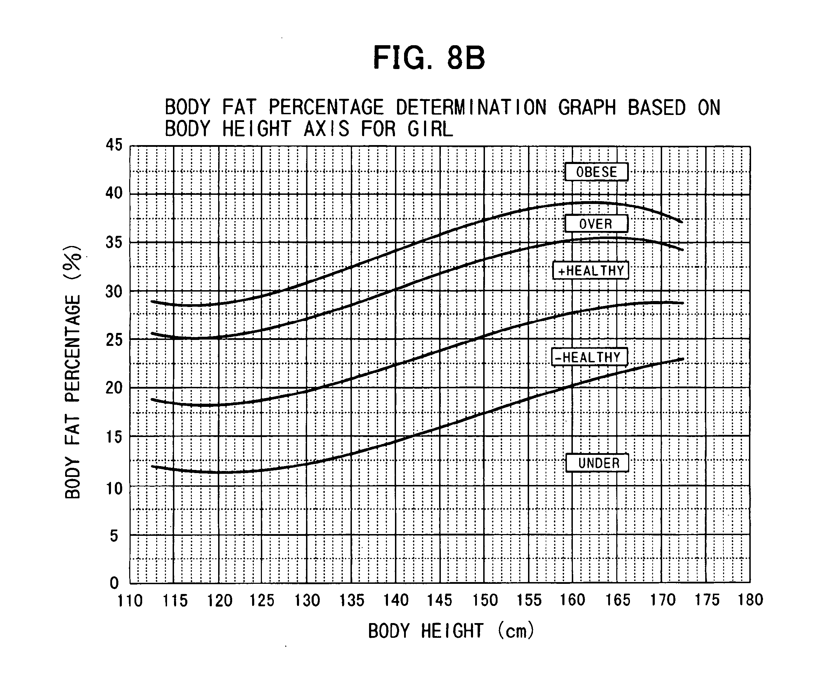

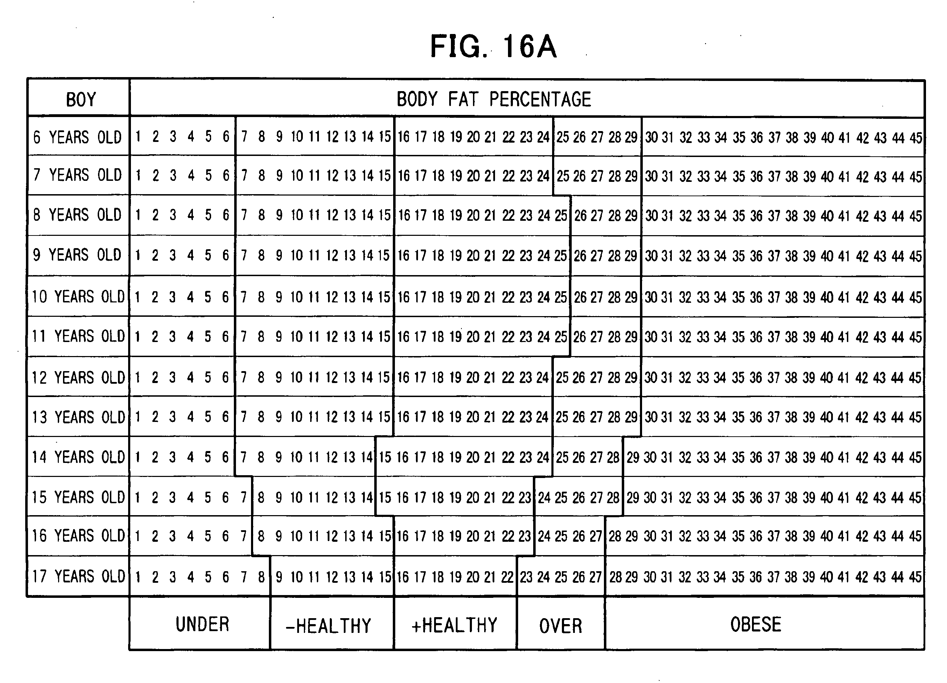

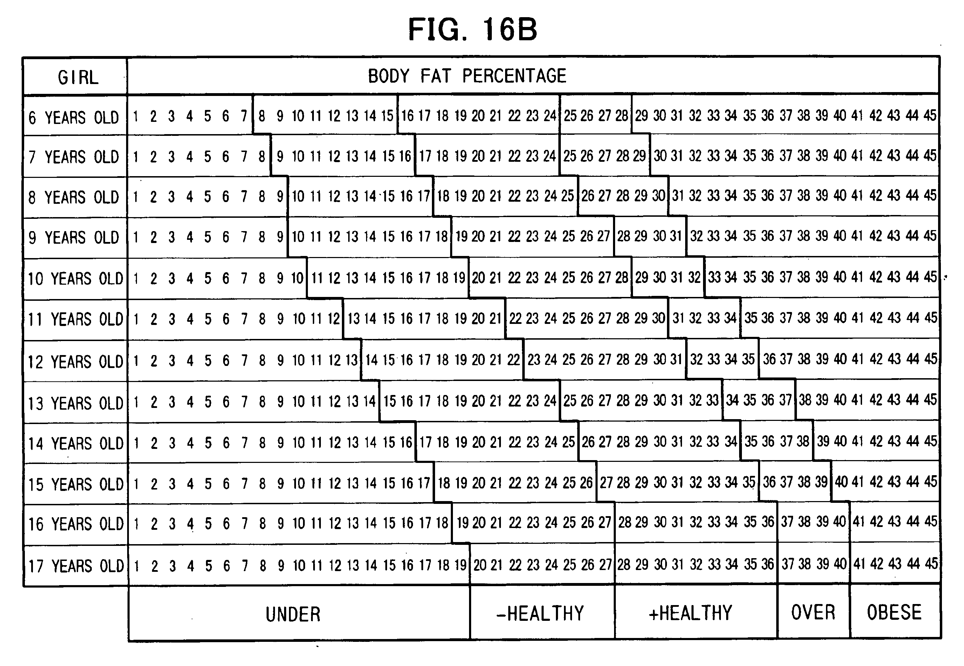

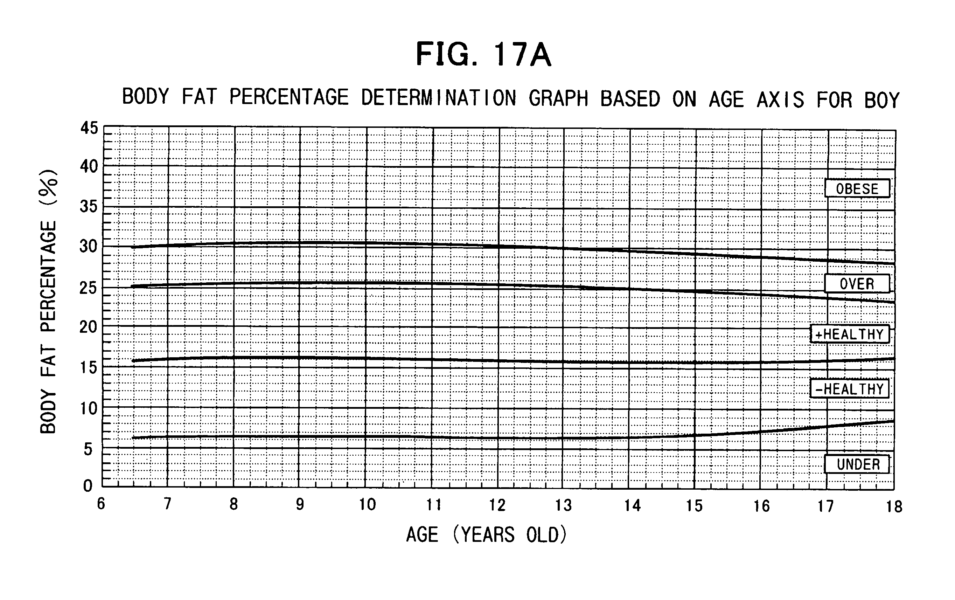

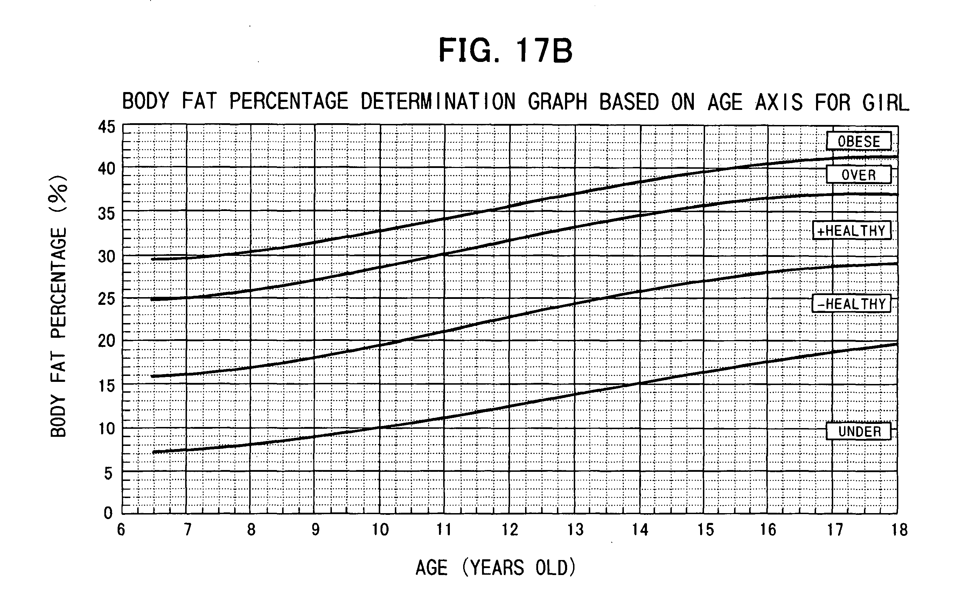

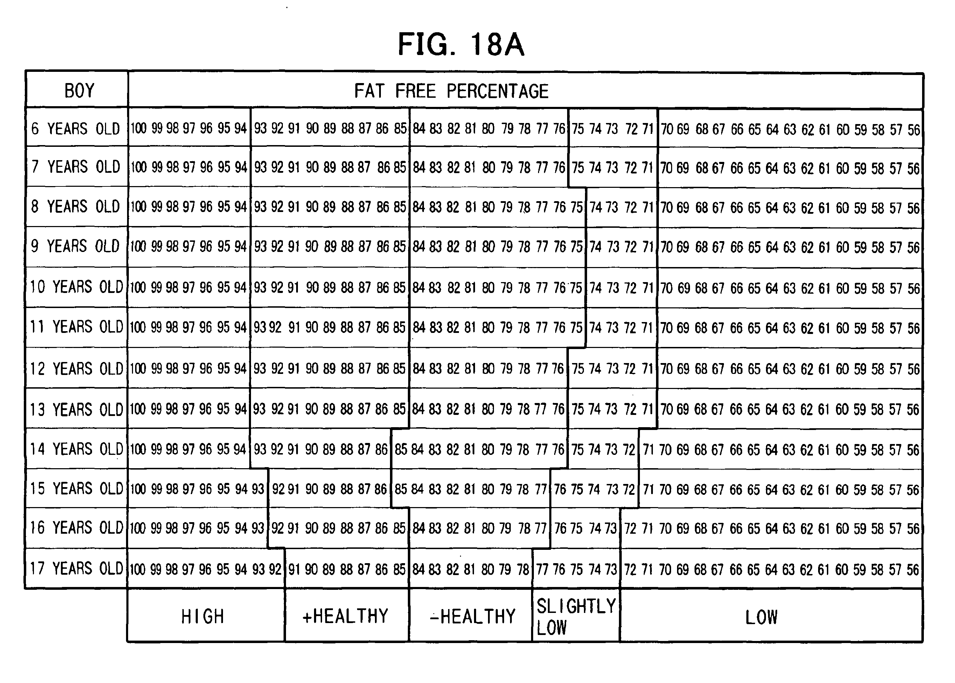

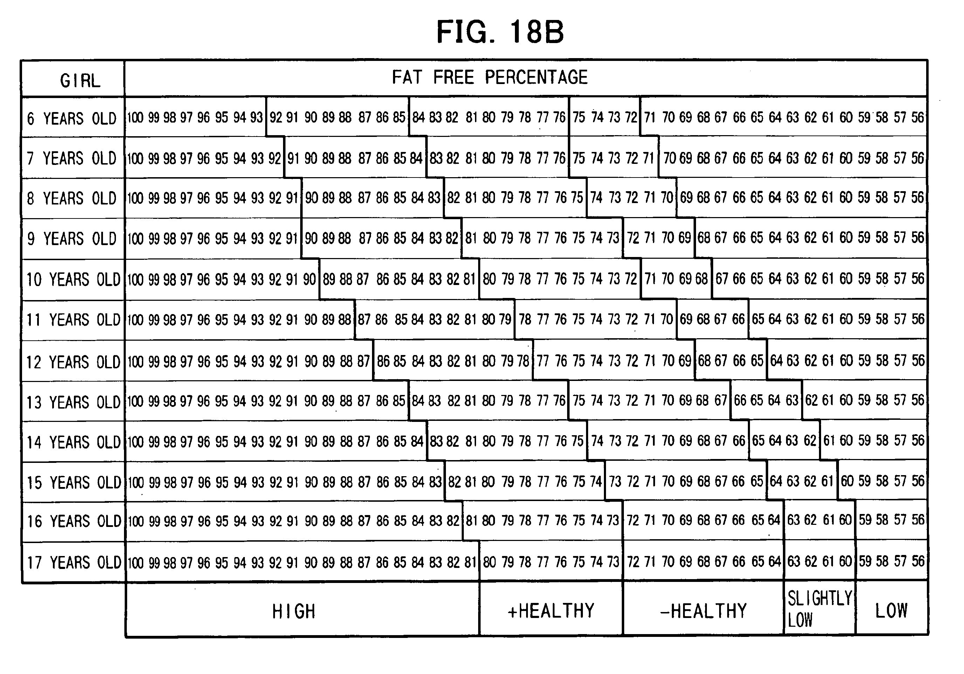

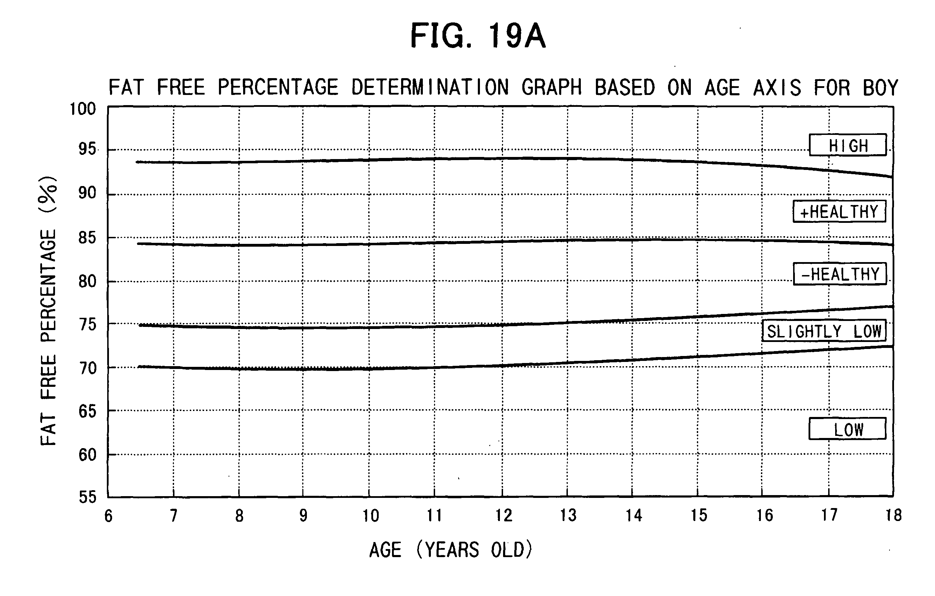

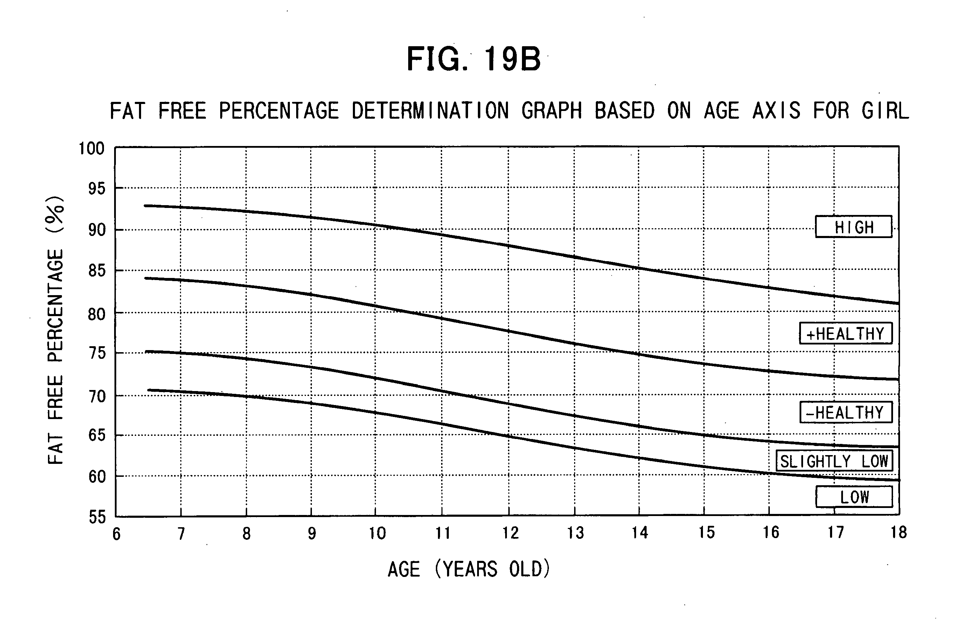

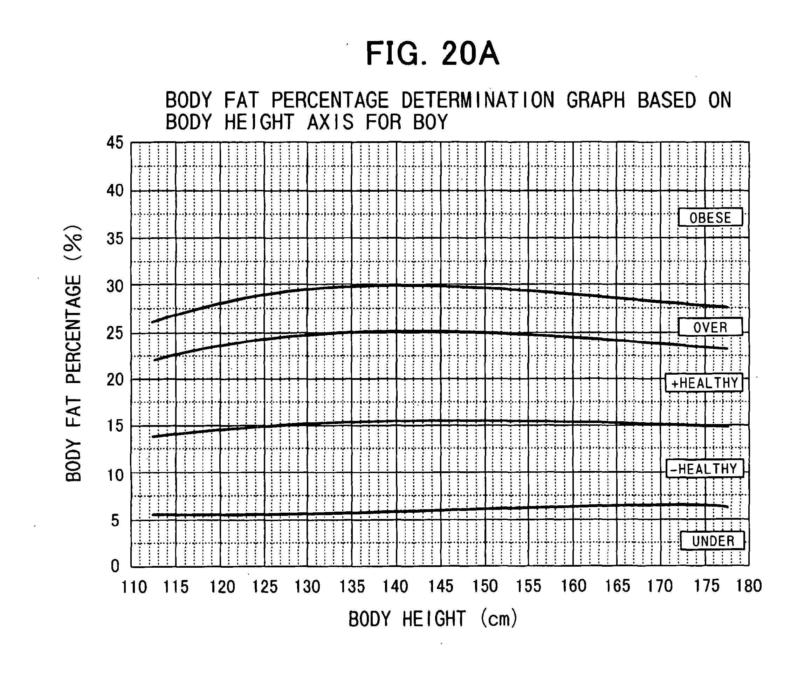

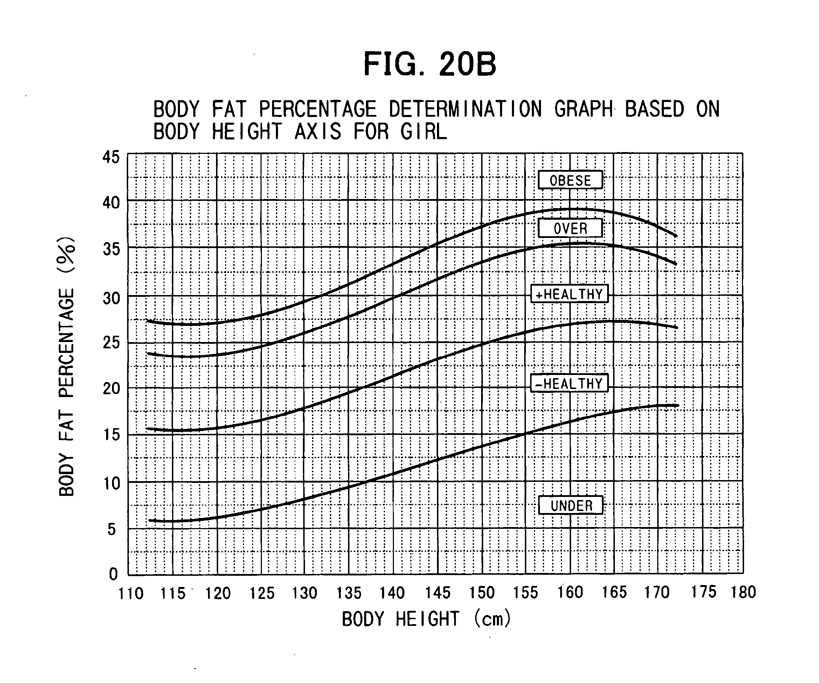

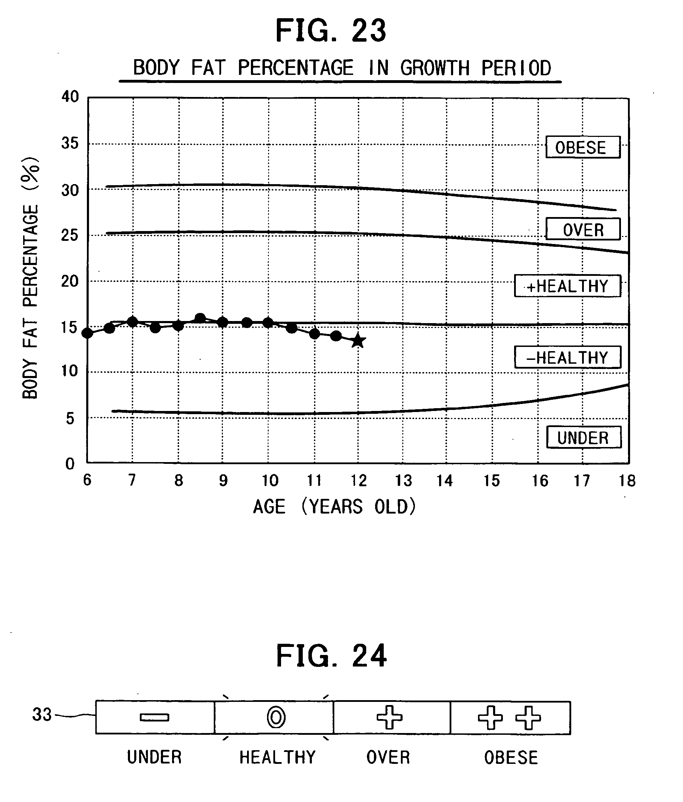

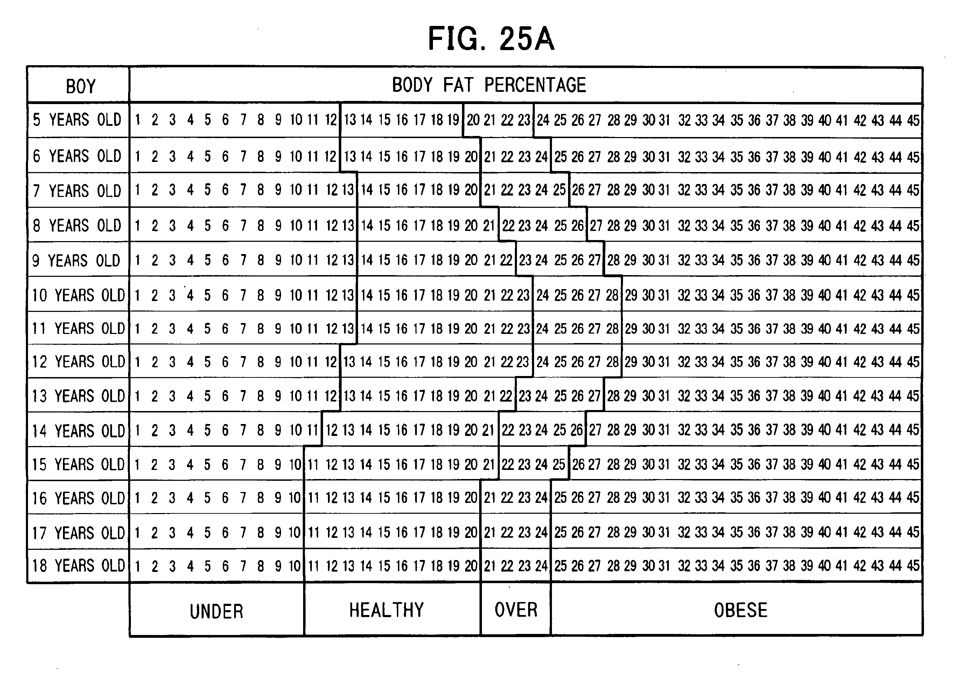

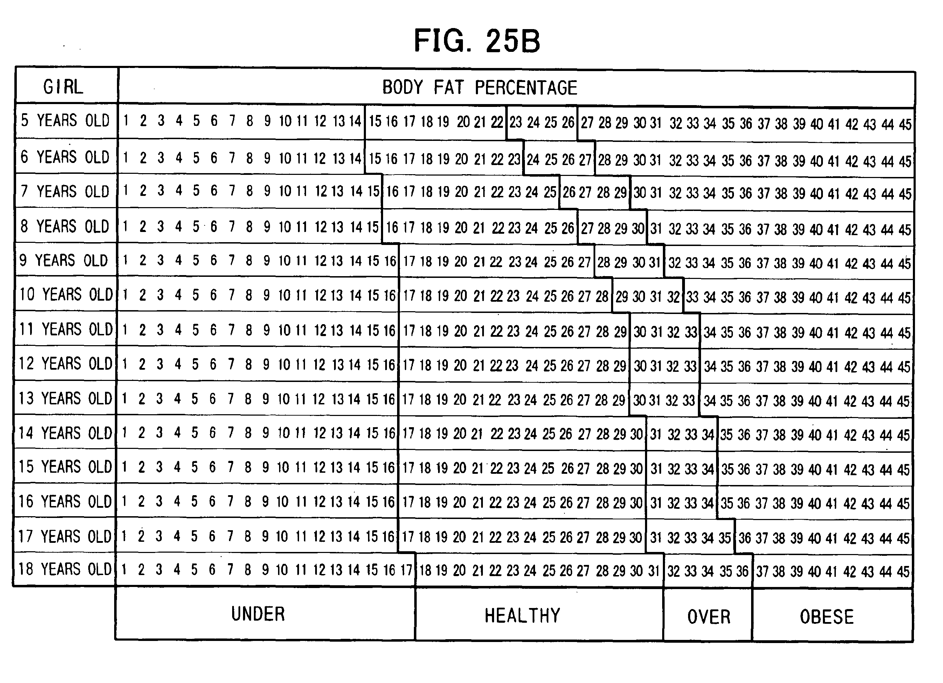

Further, in the above example, as the body composition determination reference tables, the body fat percentage determination reference tables (for Japanese) shown in Fig. 4 and the fat free percentage determination reference tables (for Japanese) shown in Fig. 6 are used. It is also possible to use body fat percentage determination reference tables (for Japanese) shown in Fig. 16, fat free percentage determination reference tables (for Japanese) shown in Fig. 18, and body fat percentage determination reference tables (for Westerners) shown in Fig. 25. Further, in the above example, as the determination graph data, the body fat percentage determination graph data shown in Fig. 5, the fat free percentage determination graph data shown in Fig. 7 and the body fat percentage determination graph data shown in Fig. 8 are used. It is also possible to use body fat percentage determination graph data shown in Fig. 17, fat free percentage determination graph data shown in Fig. 19 and body fat percentage determination graph data shown in Fig. 20. Further, the bar graph (LED 33) shown in Fig. 15 may have symbols which cause a subject to image proper grades (under, healthy, over, obese) for a body fat percentage as shown in Fig. 24.

Next, a description will be given to the reason why the body composition determination reference tables (Figs. 16 and 18) and the determination graph data (Figs. 17, 19 and 20) have different borderlines from those of the body composition determination reference tables (Figs. 4 and 6) and the determination graph data (Figs. 5, 7 and 8).

As in the case of the body composition determination reference tables (Figs. 4 and 6) and the determination graph data (Figs. 5, 7 and 8), the body composition determination reference tables (Figs. 16 and 18) and the determination graph data (Figs. 17, 19 and 20) show proper grades (under, -healthy, +healthy, over, obese) for a value associated with the body composition (body fat percentage or fat free percentage) of children as a plurality of ranges according to an indicator (age or body height) indicating the level of the growth of children based on an obesity determination standard (which determines a child with a degree of obesity of +50% or higher to be highly obese, a child with a degree of obesity of not lower than +30% and lower than +50% to be moderately obese, a child with a degree of obesity of not lower than +20% and lower than +30% to be over, a child with a degree of obesity of not lower than -20% and lower than +20% to be healthy, and a child with a degree of obesity of lower than -20% to be under) showing proper grades for the degree of obesity (= (body weight (kg) - standard body weight (kg))/standard body weight (kg) x 100) of children as a plurality of ranges.

More specifically, the body composition determination reference tables and the determination graph data are prepared as follows. First, values (body fat percentage and fat free percentage) associated with body composition and the degree of obesity are measured for each of a number of children. Then, for each sex, correlation equations between the values associated with body composition and the degree of obesity are determined according to indicators (age and body height) indicating the level of the growth of children (at intervals of 1 age or at intervals of 5 cm in body height). Then, from these correlation equations, values associated with body composition which correspond to the obesity degree borderlines (+50%, +30%, +20%, -20%) of the obesity determination standard are determined. Then, for each sex, these determined values associated with body composition according to the indicators indicating the level of the growth of children are plotted on axes representing the relationships between the values associated with body composition and the indicators indicating the level of the growth of children so as to prepare graphs. The thus obtained graph data are the determination graph data, and tables prepared from the determination graph data are the body composition determination reference tables.

In such a process, when the body composition determination reference tables (Figs. 16 and 18) and the determination graph data (Figs. 17, 19 and 20) are determined, the values associated with body composition are determined to have higher estimation accuracy than when the body composition determination reference tables (Figs. 4 and 6) and the determination graph data (Figs. 5, 7 and 8) are determined. This is the reason why they have different borderlines.

## Claims

1. A child body composition meter having a function of determining the body composition of children, comprising:
- a platform (27) having current passing electrodes (13) and measuring electrodes (14) on a top surface thereof, adopted to make contact with the bottoms of children's feet, said electrodes (13, 14), together with a current supplying circuit (12) and a voltage detecting circuit (15) forming a bioelectrical impedance measuring section (11), said bioelectrical impedance measuring section (11) and a microcomputer (7) constitute a means for acquiring a child body composition,
- a body weight measuring section (21) adapted to measure a body weight of a child standing on the platform (27),
- a setting key (3), an alteration key (4) and a graph switching key (5) for setting, altering and displaying data on a display section (31) provided on the top surface of the platform (27),
- said microcomputer (7) forming part of a means (3, 4, 7) for acquiring a child growth indicator and forming a child body composition determining means and a child body determination standard storing means,
- said microcomputer (7) being programmed to:
determine a proper grade of a value associated with the body composition of a child based on said stored child body determination standards as a plurality of ranges of the body composition according to said child growth indicator, and
- a determination result displaying means (7, 31) consisting of said display section (31) and said microcomputer (7) adapted to display said proper grade of said value associated with said child's body composition.

2. A child body composition meter according to claim 1, **characterized in that** the indicator indicating the level of growth of the child is age and/or body height.

3. A child composition meter as claimed in claims 1 or 2, **characterized by** said child body determination standards comprising said plurality of ranges are stored in said child body determination standard storing means as reference tables containing a plurality of values for each of said plurality of ranges.

4. A child body composition meter as claimed in one of the preceding claims 1 to 3, **characterized in that** said value associated with the child body composition is a body fat percentage, a fat-free percentage or a muscle percentage.

5. A child body composition meter as claimed in claim 4, **characterized in that** said plurality of ranges, in terms of body fat percentage, fat-free percentage or muscle percentage are defined as "high", "+ healthy", " - healthy", "slightly low" and "low", or "under", -healthy, + healthy, "over" and "obese".

6. A child body composition meter as claimed in claim 1, **characterized by** a determination object selecting means consisting of a setting key (3), the alteration key (4) and said microcomputer (7), wherein the determination object selecting means is adapted to select said value associated with the body composition of the child acquired by the body composition acquiring means (7,11).

7. A child body composition meter as claimed in claims 1 or 6, **characterized in that** said determination result displaying means (7,31) is adapted to display history graphs, said history graphs each comprising a time axis and a body composition value axis, the history graphs representing a current value associated with the body composition of the child together with past values associated with the body composition of the child such that the current value is positioned in the center of the time axis,
one history graph showing a transition of the values of weeks,
another history graph showing the transition of the values by months,
the other history graph showing the transition of the values by years.

8. A child body composition meter as claimed in claim 7, **characterized by** an abrupt change warning means comprising the display section (31) and the microcomputer (7) adapted to give a warning when the amount of change between the current value associated with the body composition of the child acquired by the body composition acquiring means and the past values associated with the body composition of the child is larger than a predetermined range value.

9. A child body composition meter as claimed on one of the preceding claims 3 to 8, **characterized in that** the body composition determination standards shows proper grades for values associated with the body composition of children as a plurality of ranges at an interval of one year.

10. A child body composition meter as claimed in one of the preceding claims 3 to 8, **characterized in that** the body composition determination standard shows proper grades for values associated with the body composition of children as a plurality of ranges at intervals of 5 cm in body height.

11. A child body composition meter as claimed in one of the preceding claims 1 to 10, **characterized in that** the value associated with the child body composition is the degree of obesity.

12. A child body composition meter as claimed in one of the preceding claims 1 to 10, **characterized in that** the value associated with the child body composition is a BMI.

13. A child body composition meter as claimed in one of the preceding claims 1 to 10, **characterized in that** the value associated with the child body composition is a Rohrer index.

## Patentansprüche

1. Kinderkörper- Zusammensetzungsmessgerät, mit einer Funktion der Bestimmung der Körperzusammensetzung von Kindern, aufweisend:
- eine Plattform (27) mit Stromdurchleitungselektroden (13) und Messelektroden (14) an einer Oberseite derselben, vorgesehen, um mit den Unterseiten der Füße des Kindes einen Kontakt herzustellen, wobei die Elektroden (13, 14), gemeinsam mit einem Stromzuführungsschaltkreis (12) und einem Spannungserfassungsschaltkreis (15) einen bioelektrischen Impedanz- Messabschnitt(11) bilden, wobei der bioelektrische Impedanz- Messabschnitt(11) und ein Mikrocomputer(7) eine Einrichtung zum Erlangen einer Kinderkörper-Zusammensetzung bilden,
- einen Körpergewicht- Messabschnitt (21), vorgesehen ein Körpergewicht eines Kindes , das auf einer Plattform (27) steht, zu messen,
- einen Festlegungsschlüssel (3), einen Änderungsschlüssel (4) und einen Diagramm- Umschaltschlüssel (5) zum Festlegen, Ändern und Anzeigen von Daten auf einem Anzeigeabschnitt (31), vorgesehen auf der Oberseite der Plattform (27),
- wobei der Mikrocomputer (7) einen Teil einer Einrichtung (3, 4, 7) zum Erlangen eines Kinderwachstumsindikators bildet, und der eine Kinderkörper- Zusammensetzungs- Bestimmungseinrichtung und eine Kinderkörperbestimmungs- Standardspeichereinrichtung bildet,
- wobei der Mikrocomputer (7) programmiert ist, zum:
Bestimmen eines richtigen Grades eines Wertes, der der Kinderkörperzusammensetzung zugehörig ist, auf der Grundlage der gespeicherten Kinderkörper- Bestimmungsstandards als einer Mehrzahl von Bereichen der Körperzusammensetzung entsprechend dem Kinderwachstumsindikator, und
- einer Bestimmungsergebnis- Anzeigeeinrichtung (7, 21), bestehend aus dem Anzeigeabschnitt (31) und dem Mikrocomputer (7), vorgesehen, den richtigen Grad des Wertes, der der Körperzusammensetzung des Kindes zugehörig ist, anzuzeigen.

2. Kinderkörper- Zusammensetzungsmessgerät nach Anspruch 1, **dadurch gekennzeichnet, dass** der Indikator, das Maß des Wachstums des Kindes anzeigt, das Alter und / oder die Körpergröße ist.

3. Kinderkörper- Zusammensetzungsmessgerät nach Anspruch 1 oder 2, **gekennzeichnet dadurch, dass** die Kinderkörper- Bestimmungsstandards eine Mehrzahl von Bereichen aufweisen, die in der Kinderkörperbestimmungsstandard- Speichereinrichtung als Referenztabellen gespeichert sind, die eine Mehrzahl von Werten für jeden der Mehrzahl von Bereichen aufweisen.

4. Kinderkörper- Zusammensetzungsmessgerät nach einem der vorhergehenden Ansprüche 1 bis 3, **dadurch gekennzeichnet, dass** der zu der Kinderkörper- Zusammensetzung zugehörige Wert ein Körperfettprozentsatz, ein Fettfreier Prozentsatz oder ein Muskelprozentsatz ist.

5. Kinderkörper- Zusammensetzungsmessgerät nach Anspruch 4, **dadurch gekennzeichnet, dass** die Mehrzahl der Bereiche im Hinblick auf den Körperfettprozentsatz, den Fettfreien Prozentsatz oder den Muskelprozentsatz als "hoch", "+ gesund", "- gesund", "leicht niedrig" und "niedrig", oder "unter", - gesund, + gesund, "über" und "fettleibig" bestimmt werden.

6. Kinderkörper- Zusammensetzungsmessgerät nach Anspruch 1, **gekennzeichnet durch** eine Bestimmungsobjekt- Auswahleinrichtung, bestehend aus einem Festlegungsschlüssel (3), den Änderungsschlüssel (4) und dem Mikrocomputer (7), wobei die Bestimmungsobjekt- Auswahleinrichtung vorgesehen ist, den Wert auszuwählen, der der Körperzusammensetzung des Kindes, erlangt **durch** die Körperzusammensetzungs- Erlangungseinrichtung (7, 11), zugehörig ist.

7. Kinderkörper- Zusammensetzungsmessgerät nach Anspruch 1 oder 6, **dadurch gekennzeichnet, dass** die Bestimmungsergebnis- Anzeigeeinrichtung (7, 31) vorgesehen ist, Entwicklungsdiagramme anzuzeigen, wobei die Entwicklungsdiagramme jeweils eine Zeitachse und eine Achse der Körperzusammensetzung aufweisen,
wobei die Entwicklungsdiagramme einen Momentanwert, zugehörig der Körperzusammensetzung des Kindes, gemeinsam mit vergangenen Werten, zugehörig der Körperzusammensetzung des Kindes repräsentieren derart, dass der Momentanwert in der Mitte der Zeitachse positioniert ist,
wobei ein Entwicklungsdiagramm einen Übergang der Werte nach Wochen zeigt, ein weiteres Entwicklungsdiagramm den Übergang der Werte nach Monaten zeigt,
das andere Entwicklungsdiagramm den Übergang der Werte nach Jahren zeigt.

8. Kinderkörper- Zusammensetzungsmessgerät nach Anspruch 7, **gekennzeichnet durch** eine Warneinrichtung bei einer abrupten Veränderung, aufweisend den Anzeigeabschnitt (31) und den Mikrocomputer (7), vorgesehen, um eine Warnung zu geben, wenn die Größe der Veränderung zwischen dem Momentanwert, zugehörig der Körperzusammensetzung des Kindes, erlangt **durch** die Körperzusammensetzung- Erlangungseinrichtung, und den vergangenen Werten, zugehörig der Körperzusammensetzung des Kindes, größer als ein vorbestimmter Bereichswert ist.

9. Kinderkörper- Zusammensetzungsmessgerät nach einem der vorhergehenden Ansprüche 3 bis 8, **dadurch gekennzeichnet, dass** die Körperzusammensetzungs- Bestimmungsstandards richtige Grade für die Werte, zugehörig der Körperzusammensetzung der Kinder, als eine Mehrzahl von Bereichen in einem Intervall von einem Jahr zeigen.

10. Kinderkörper- Zusammensetzungsmessgerät nach einem der vorhergehenden Ansprüche 3 bis 8, **dadurch gekennzeichnet, dass** die Körperzusammensetzungs- Bestimmungsstandards richtige Grade für Werte, zugehörig der Körperzusammensetzung der Kinder, als eine Mehrzahl von Bereichen in Intervallen von 5 cm in der Körpergröße zeigen.

11. Kinderkörper- Zusammensetzungsmessgerät nach einem der vorhergehenden Ansprüche 1 bis 10, **dadurch gekennzeichnet, dass** der Wert, zugehörig der Körperzusammensetzung des Kindes, der Grad der Fettleibigkeit ist.

12. Kinderkörper- Zusammensetzungsmessgerät nach einem der vorhergehenden Ansprüche 1 bis 10, **dadurch gekennzeichnet, dass** der Wert, zugehörig der Körperzusammensetzung des Kindes, ein BMI (Body Mass Index) ist.

13. Kinderkörper- Zusammensetzungsmessgerät nach einem der vorhergehenden Ansprüche 1 bis 10, **dadurch gekennzeichnet, dass** der Wert, zugehörig der Körperzusammensetzung des Kindes, ein Rohrer- Index ist.

## Revendications

1. Appareil de mesure de la composition corporelle d'enfant ayant une fonction de détermination des compositions corporelles d'enfants, comprenant :
- une plateforme (27) comportant des électrodes de passage de courant (13) et des électrodes de mesure (14) sur une surface supérieure de celle-ci, adaptée pour venir en contact avec les surfaces inférieures des pieds d'enfants, lesdites électrodes (13, 14), avec un circuit d'alimentation en courant (12) et un circuit de détection de tension (15), formant une section de mesure d'impédance bioélectrique (11), ladite section de mesure d'impédance biolélectrique (11) et un micro-ordinateur (7) constituant des moyens pour acquérir une composition corporelle d'enfant,
- une section de mesure de poids corporel (21) adaptée pour mesurer un poids corporel d'un enfant se trouvant sur la plateforme (27),
- une touche de réglage (3), une touche de modification (4) et une touche de changement de graphe (5) pour régler, modifier et afficher des données sur une section d'affichage (31) prévue sur la surface supérieure de la plateforme (27),
- ledit micro-ordinateur (7) faisant partie de moyens (3, 4, 7) pour acquérir un indicateur de croissance d'enfant et formant des moyens de détermination de composition corporelle d'enfant et des moyens de mémorisation de normes de détermination corporelle d'enfant,
- ledit micro-ordinateur (7) étant programmé pour :
déterminer un niveau correct d'une valeur associée à la composition corporelle d'un enfant sur la base desdites normes de détermination corporelle d'enfant mémorisées en tant que pluralité de plages de la composition corporelle conformément audit indicateur de croissance d'enfant, et
- des moyens d'affichage de résultat de détermination (7, 31) consistant en ladite section d'affichage (31) et ledit micro-ordinateur (7) adaptés pour afficher ledit niveau approprié de ladite valeur associée à ladite composition corporelle de l'enfant.

2. Appareil de mesure de composition corporelle d'enfant selon la revendication 1, **caractérisé en ce que** l'indicateur indiquant le niveau de croissance de l'enfant est l'âge et/ou la hauteur du corps.

3. Appareil de mesure de composition corporelle d'enfant selon les revendications 1 ou 2, **caractérisé en ce que** lesdites normes de détermination corporelle d'enfant comprenant ladite pluralité de plages sont mémorisées dans lesdits moyens de mémorisation de normes de détermination corporelle d'enfant en tant que tables de référence contenant une pluralité de valeurs pour chacune de ladite pluralité de plages.

4. Appareil de mesure de composition corporelle d'enfant selon l'une des revendications précédentes 1 à 3, **caractérisé en ce que** ladite valeur associée à la composition corporelle d'enfant est un pourcentage de graisse corporelle, un pourcentage sans graisse ou un pourcentage de muscles.

5. Appareil de mesure de composition corporelle d'enfant selon la revendication 4, **caractérisé en ce que** ladite pluralité de plages, en termes de pourcentage de graisse corporelle, de pourcentage sans graisse ou de pourcentage de muscles, sont définies en tant que « hauteur », « + en bonne santé », « - en bonne santé », « légèrement bas » et « bas », ou « inférieur », « - en bonne santé », « + en bonne santé », « supérieur » et « obèse ».

6. Appareil de mesure de composition corporelle d'enfant selon la revendication 1, **caractérisé par** des moyens de sélection d'objet de détermination consistant en une touche de réglage (3), la touche de modification (4) et ledit micro-ordinateur (7), dans lequel les moyens de sélection d'objet de détermination sont adaptés pour sélectionner ladite valeur associée à la composition corporelle de l'enfant acquise par les moyens d'acquisition de composition corporelle (7, 11).

7. Appareil de mesure de composition corporelle d'enfant selon les revendications 1 ou 6, **caractérisé en ce que** lesdits moyens d'affichage de résultat de détermination (7, 31) sont adaptés pour afficher des graphes d'historique, lesdits graphes d'historique comprenant chacun un axe de temps et un axe de valeur de composition corporelle,
les graphes d'historique représentant une valeur actuelle associée à la composition corporelle de l'enfant avec les valeurs passées associées à la composition corporelle de l'enfant de sorte que la valeur actuelle soit positionnée au centre de l'axe de temps,
un graphe d'historique montrant une transition des valeurs par semaines,
un autre graphe d'historique montrant la transition des valeurs par mois,
l'autre graphe d'historique montrant la transition des valeurs par années.

8. Appareil de mesure de composition corporelle d'enfant selon la revendication 7, **caractérisé par** des moyens d'avertissement de changement brutal comprenant la section d'affichage (31) et le micro-ordinateur (7) adaptés pour donner un avertissement lorsque la quantité de changement entre la valeur actuelle associée à la composition corporelle de l'enfant acquise par les moyens d'acquisition de composition corporelle et les valeurs passées associées à la composition corporelle de l'enfant est supérieure à une valeur de plage prédéterminée.

9. Appareil de mesure de composition corporelle d'enfant selon l'une des revendications précédentes 3 à 8, **caractérisé en ce que** les normes de détermination de composition corporelle montrent des niveaux corrects pour les valeurs associées à la composition corporelle d'enfants en tant que pluralité de plages à un intervalle d'un an.

10. Appareil de mesure de composition corporelle d'enfant selon l'une des revendications précédentes 3 à 8, **caractérisé en ce que** les normes de détermination de composition corporelle montrent des niveaux corrects pour les valeurs associées à la composition corporelle d'enfants en tant que pluralité de plages à des intervalles de 5 cm de hauteur du corps.

11. Appareil de mesure de composition corporelle d'enfant selon l'une des revendications précédentes 1 à 10, **caractérisé en ce que** la valeur associée à la composition corporelle d'enfant est le degré d'obésité.

12. Appareil de mesure de composition corporelle d'enfant selon l'une des revendications précédentes 1 à 10, **caractérisé en ce que** la valeur associée à la composition corporelle d'enfant est un IMC.

13. Appareil de mesure de composition corporelle d'enfant selon l'une des revendications précédentes 1 à 10, **caractérisé en ce que** la valeur associée à la composition corporelle d'enfant est un indice de Rohrer.
